# NEW EUROPEAN PATENT SPECIFICATION

(11) **EP 1 420 845 B2**
(45) Date of publication and mention of the opposition decision: **09.12.2015**
(45) Mention of the grant of the patent: 12.03.2008
(21) Application number: 02754547.4
(22) Date of filing: 28.06.2002
(51) Int. Cl.: A61M 25/00

(54) **A METHOD OF PRODUCING A CATHETER AND A CATHETER**
KATHETER UND METHODE ZUM HERSTELLEN EINES KATHETERS
PROCEDE DE FABRICATION DE CATHETER ET CATHETER

(30) Priority: 29.06.2001 DK 200101041; 29.06.2001 US 893514; 24.09.2001 DK 200101386; 13.12.2001 DK 200101869; 13.12.2001 DK 200101870; 27.12.2001 US 26819; 17.04.2002 DK 200200569; 17.04.2002 DK 200200570; 13.06.2002 DK 200200895
(43) Date of publication of application: 26.05.2004
(60) Divisional application: 08152518.0
(73) Proprietor: Coloplast A/S, 3050 Humlebaek (DK)
(72) Inventor: TANGHOJ, Allan, DK-2980 Kokkedal (DK); JENSEN, Lars, Bogelund, DK-2610 Rodovre (DK)
(74) Representative: Inspicos P/S
(86) International application number: PCT/DK2002/000451
(87) International publication number: WO 2003/002325

(56) References cited:
- EP-A- 0 824 930
- EP-A- 0 925 802
- EP-A1- 0 980 892
- EP-A1- 1 034 811
- EP-B1- 0 958 911
- EP-B1- 1 116 567
- WO-A-90/00960
- WO-A1-00/16843
- WO-A1-01/32240
- WO-A1-96/19254
- WO-A1-97/07707
- DE-C- 4 140 099
- DE-U1- 20 007 733
- GB-A- 2 230 702
- US-A- 1 524 704
- US-A- 3 865 666
- US-A- 3 894 540
- US-A- 3 901 965
- US-A- 3 947 175
- US-A- 4 041 122
- US-A- 4 284 459
- US-A- 4 750 877
- US-A- 4 957 682
- US-A- 5 167 647
- US-A- 5 510 065
- US-A- 6 059 107
- US-A- 6 063 063
- US-A- 6 149 996
- US-A- 6 158 912
- See Citation sheet and the decision, page 2

## Description

### Field of the invention

The present invention relates to a method of producing a medical catheter. In particular, the invention relates to a method wherein a catheter is produced by solidifying a fluid catheter material in a mould.

### Background of the invention

In general, medical catheters are used for draining bodily fluids such as blood and urine. A catheter for medical use is typically provided with a tubular oblong catheter body part made from a piece of flexible medical hose with an internal conduit, e.g. a PVC or PU hose having a substantially circular outer and inner cross-sectional shape. In an insertable end thereof, the catheter forms one or more openings through which fluid can drain from a bodily cavity and into the tubular body. In order to ease the insertion and to avoid injuring the bodily tissue when the catheter is inserted into a body opening and guided through a bodily canal, e.g. the urethra or a blood vessel, the insertable end is normally provided with a smoothly rounded tip. In the case of most catheters, the tip is formed by heating and melting an end part of the medical hose until its conduit seals. Even though the existing catheters are formed with a tip providing an acceptably safe and comfortable insertion, it is a desire to further shape a larger and more curved tip of the inserted part of the catheter since this may allow for an easier and safer insertion. However, the present technique of forming the tip of a medial hose does not support in making such a tip. As an alternative, some catheters are made from a medical hose with a glued-on catheter tip. This solution allows for a catheter with a larger and more curved tip, but the additional process step of providing a tip part and gluing the part to the medical hose implies additional production costs. Moreover, there is potential risk that the tip falls off during the catheterisation and thus remains inside the body cavity, e.g. in the bladder. For economical and for safety reasons, the on-glued catheter tip is therefore undesired. US 4,041,122 discloses a process for the molding of hollow articles such as full length Foley catheters. US 3,865,666 discloses the step of moulding a balloon to a shaft to provide a balloon catheter. The shaft is made in a continuous extrusion process.

When the tip has been formed, a number of draining holes are normally drilled or punched radially into the hose in the vicinity of the tip. In order to avoid that the holes damage the bodily tissue during the insertion of the catheter, the edges of the holes must be smoothly rounded. Accordingly, the process of making the holes is time-consuming and expensive.

At its opposite end, the oblong catheter body is formed with an opening allowing the fluid to drain out of the catheter and into an appropriate place of disposal. In this opposite end, most catheters are provided with a connector part. The connector part allows the catheter to be connected e.g. to a bag for collecting the fluid. The connector part is normally formed by adhesively bonding a funnel-shaped member to the medical hose. Once again, the additional process of gluing a separate member onto the hose is cost-inefficient and implies a larger percentage of defect products.

### Description of the invention

It is an object of the present invention to overcome the above described problems, in particular with respect to providing a strong adherence between two different materials in a catheter. Accordingly, the invention provides a method according to claim 1.

The injection may take place in a regular machine for injection moulding. Depending upon the size and length of the catheter, the injection pressure may be In the range of 500-1500 bar, such as 750-1250 bar such as in the size of 1000 bar.

The injection moulding process is in particular suitable for relatively short catheters, i.e. catheters which are in the range of 50-90 mm., such as in the range of 55-85 mm., such as in the range of 60-80 mm. such as with a length in the size of 70 mm. which length has been found to be a suitable insertable length for most female individuals. For male individuals, catheter sections may preferably be provided in a length in the range of 180-250 mm., such as in the range of 190-240 mm., such as in the range of 200-230 mm. such as in the size of 220 mm.

The catheter may further comprise connection means for connecting the proximal insertion section to a further catheter section or to a urinary collection bag. The connector part may be made from the same material as the proximal insertion section, whereby, at the step of forming the proximal insertion section, the proximal insertion section and the connector part may be formed substantially simultaneously. Alternatively, the connector part may be made from a material different from the material of the proximal insertion section, whereby the connector part and the proximal insertion section are formed in distinct process steps, for example in a multi-component Injection moulding process.

The mould could be formed to define the body part as an oblong hollow, tubular part with an internal conduit of a size allowing bodily fluid to be drained through the catheter body.

As an alternative, the body part could be provided in the form of an oblong solid kernel with one or more vanes extending radially from the kernel and along the entire length thereof. The vanes thus define a number of draining passages for draining urine between the kernel and a bodily draining passage, e.g. the urethra.
In order to allow the bodily fluid, e.g. urine from the bladder, to enter the hollow, tubular body part of the catheter, the mould could be formed to define at least one draining hole in the vicinity of the tip.

A connector part may be provided for connecting the catheter to a hose for extending the length of the catheter or for connecting the catheter to disposal means, e.g. to a urinary collection bag. The connector section could preferably be made in one part with the catheter body and the tip. The connector part may be made from the same material as the proximal Insertion section and preferably substantially simultaneous therewith. Alternatively, the connector part may be made from a material different from the material of the proximal insertion section. The connector part and the proximal insertion section are thus formed in distinct process steps, for example in a multi-component injection moulding process. The connector part may also be arranged as a separate component in the injection mould before the injection of the catheter material so that the connector part is moulded into engagement with the catheter body part during the injection moulding of the body part of the catheter. In a similar manner, a catheter tip may be arranged as a separate component in the mould and, during the injection moulding of the body part of the catheter, be moulded into the catheter. In a similar manner, additional components may be arranged In the mould prior to the injection moulding of the catheter body. As an example, one or more ring-shaped coloured members may be arranged for the purpose of visualising a certain length of the catheter, e.g. for visualising the intended insertable length. As another example, one or more objects made of a material, which improves the visualisation of the catheter in an x-ray or ultra-sound image may be arranged in the mould prior to the injection moulding of the catheter body.

In order to allow a user of the catheter to get a better grip, the catheter may be provided with means for attaching the catheter to peripheral articles such as a hand-grip for firmly gripping the catheter. The means for attaching the catheter to peripheral articles could be an out or inwardly extending bulge. As a further option, the catheter could be formed in one piece with means for handling the catheter during the insertion, e.g. a handle part which supports for a firm hand grip. As an example, the catheter could be provided in a diameter allowing for insertion into the urethra and with a catheter section not adapted for insertion and provided In a much larger diameter allowing a firm hand grip in the catheter.

Depending upon the type of catheterisation, the bodily fluid Is typically drained either Into a place of disposal e.g. into a toilet or into a reservoir or container for collecting the fluid. Accordingly, the mould could be formed to further define a reservoir for collecting the bodily fluids. The reservoir could be a plastic bag moulded in one piece with the catheter, e.g. by a combined injection and blow moulding process.

In order to fixate the catheter in the bodily passage, the catheter could be provided with a balloon in the vicinity of the inserted tip. The balloon could be moulded into one piece with the catheter.

Sometimes, it is desired that different parts of the catheter is provided with different characteristics. As an example, it may be desired for a urinary catheter that the insertable part is relatively soft and flexible so that the catheter can pass through the curved passage of the urethra. On the other hand, those parts of the catheter which is not adapted for insertion into the urethra may preferably be relatively less flexible, thus allowing an easier grip and allowing the inserted part of the catheter to be manipulated via the not inserted part.

Similarly, it may often be desired that the outer surface of the catheter is provided in a low-frictional material supporting an easier and more comfortable insertion of the catheter into the urinary canal. Accordingly, it may be desired to provide a surface layer of the catheter in a low-friction material such as FEP, PTFE or in a hydrophilic material such as polyvinylidone while the remaining part of the catheter is provided in a stronger and more durable material such as a thermoplastic elastomeric material, other thermoplastic materials, curable elastomeric materials, polyamide resins or elastomers or any mixture thereof, i.e. the group may comprise materials like, PVC, PU, PE, latex, and/or Kraton^{™}. As an example, the mould may be coated with a hydrophilic material prior to the injection of a thermoplastic elastomeric material into the mould. As an alternative, series of injections of one or more types of thermoplastic elastomeric materials into the mould may take place. As an example, a hydrophilic material or a similar low frictional material such as siloxane, FEP etc. may firstly be injected to form an outer layer of the catheter. Subsequently, one or more types of materials, e.g. materials with different characteristics, are injected in one or more injection cycles In order to form the rest of the catheter.

As an advantage of the injection moulding process, the surface of the catheter may easily be provided with a surface roughness defining anchoring points for adhering a low frictional material to the surface of the catheter, e.g. anchoring points for bonding a FEP or PTFE (Teflon^{™}) coating to the surface.

When injecting the material in more than one injection cycle, a catheter either comprising multiple layers or laminates, is formed. Due to the laminated structure, the laminated structure may be more durable towards mechanical stress.

The different characteristics may relate to the softness of the material after solidification, the colour of the material the strength of the material, the slipperiness of the material after solidification or to the ability of the solidified material to absorb liquid substances.

In order to reduce the resistance against insertion into the body canal, the radial size of the tip may be larger than the radial size of the rest of the catheter. As an example, the tip may be formed into an onion-shaped or bulbous knob or the tip may be formed with a conical shape. Preferably, the tip is provided with a first end part, fronting the body canal opening during the insertion and provided with a narrow radial size. From this end part, the tip widens out in an intermediate part until the radial size exceeds the radial size of the catheter body part. Between the intermediate part of the tip and the catheter body part, the radial size slopes down in a second end part until it reaches the radial size of the catheter body part.

Inlet openings for draining bodily fluid from a body cavity and into the catheter may be provided either in the first end part, in the intermediate part or in the second end part or in more than one part or even in all of the parts. As an example, the tip may be formed with a plurality of small holes in the tip.

According to a second aspect, the present invention relates to a catheter formed by the above described method, comprising a laminated structure.

### Brief description of the drawings

Preferred embodiments of the invention will now be described in details with reference to the drawing in which:
Fig. 1 shows an injection-moulded catheter with a tip and a connector,
Fig. 2 shows a bulbous catheter tip,
Fig. 3 shows a conical catheter tip,
Fig. 4 shows a spherical catheter tip,
Fig. 5 shows a catheter with a reservoir for collecting bodily fluids, and
Fig. 6 shows a catheter in the form of an oblong solid kernel with a plurality of vanes extending radially from the kernel and along the entire length thereof.

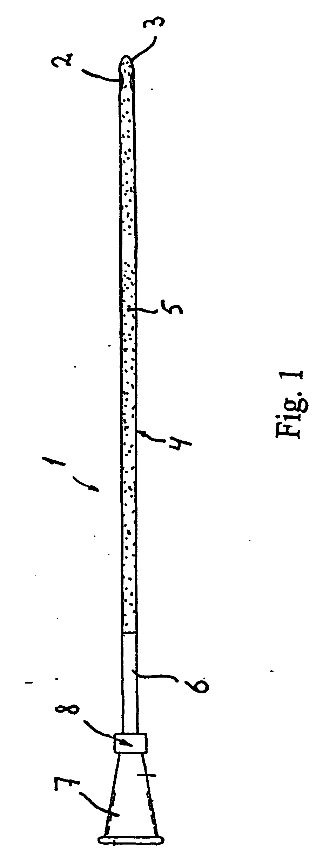

Referring to Fig. 1, the invention relates to a method of producing a catheter according to claim 1. As shown, a catheter connector section 7 may be formed In one part with the catheter body. Furthermore, means 8 for attaching the catheter to peripheral articles for easing the handling of the catheter could be formed in one part with the catheter body during the moulding process.

In the insertable end, i.e. in the vicinity of the tip, a draining hole 2 allows bodily fluids to flow from a body cavity and into an internal conduit of the catheter. The internal conduit transports the fluid to the connector part where the fluid can be disposed into a bag or into a place of disposal.

The catheter of Fig. 1, Is on its insertable part provided with a friction reducing material 5 and on its non-insertable part with a highly frictional material 6. The friction reducing material supports a safe and easy insertion of the catheter into a body canal such as the urethra and the highly frictional material supports an easier grip in the non-inserted part and thus supports for easier manipulation of the catheter. The characteristics of the materials 5 and 6, respectively, could also relate to different colours indicating to the user which part of the catheter is intended to be inserted.

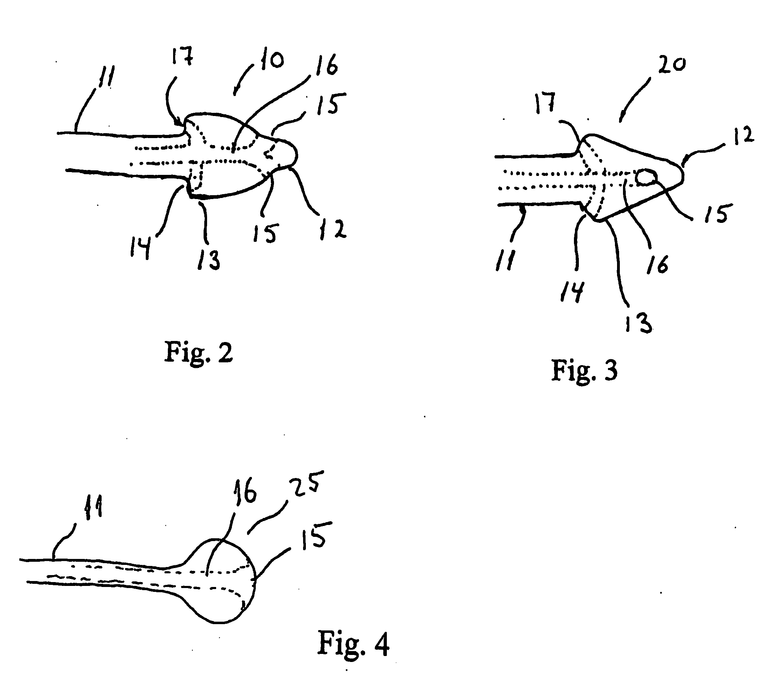

Figs. 2, 3 and 4 shows three different alternative tip shapes, i.e. a bulbous tip 10 of Fig. 2, a conical tip 20 of Fig. 3 and a spherical tip 25 of Fig. 4. The tip is provided in the inserted end of the catheter body 11 in one part with the body and during the moulding process. The tip is formed with a first end part 12 with a radial size which is smaller than the radial size of the catheter body, an intermediate part 13 with a radial size which is larger than the radial size of the catheter and a second end part 14 wherein the size slopes down to the radial size of the catheter. In order to allow bodily fluids to drain into an internal conduit of the catheter, draining holes are provided In one or more of the parts of the tip. In Figs. 2 and 3, draining holes 15 are provided in the first end part and draining holes 17 are provided in the second end part. The tip is internally moulded with a conduit 16 for guiding the fluid to the body part of the catheter. As shown in Fig. 4, a draining hole or holes may be provided in the tip, so that the opening points in the axial direction of the catheter body. In that case it is important that edges of the hole or holes are smoothly rounded in order not to injure the body canal.

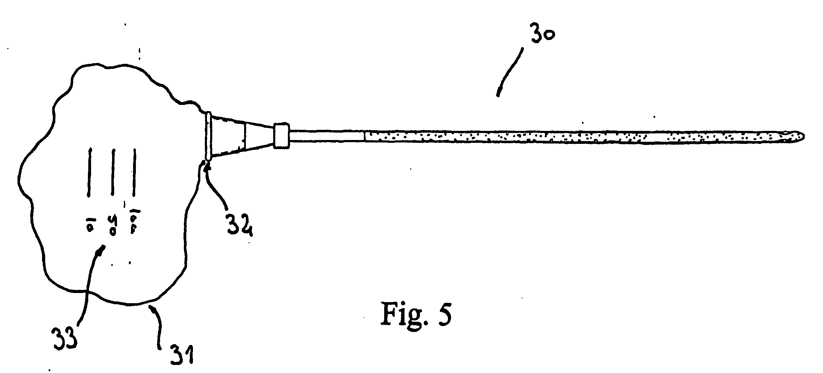

Fig. 5 shows a catheter 30 and a reservoir 31 formed by moulding in one part for collecting the bodily fluids, e.g. blood or urine. The reservoir could be formed during the injection moulding process by combining the injection moulding process with blow moulding. During this process, pressurised gas used for expanding the reservoir part of the catheter product into a plastic bag or plastic bottle shaped item. The reservoir may, during the moulding process, be provided with filling level indication marks 33 and may internally be provided with a hydrophilic material for the conversion of a liquid substance into a substantially solid or gel-like substance.

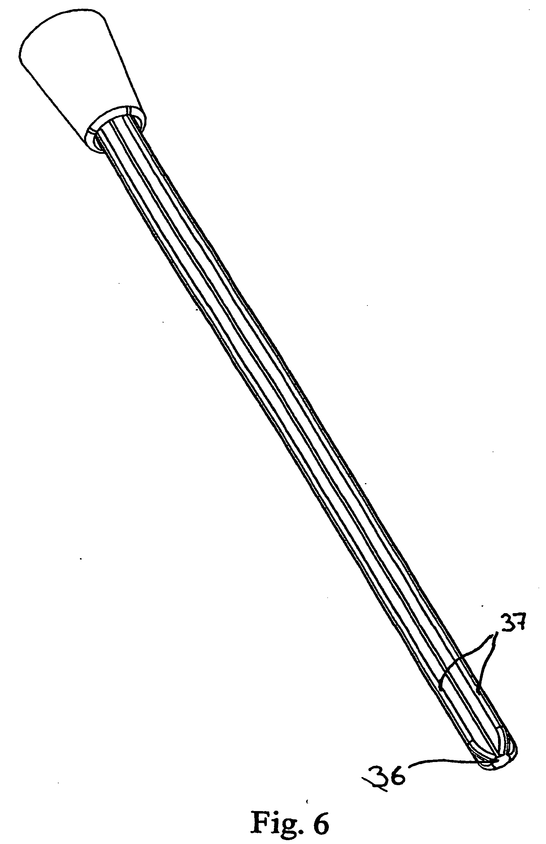

Fig. 6 shows a catheter in the form of a solid kernel 36 with a plurality of radially extending vanes 37. The vanes form a number of draining passages for draining urine between the kernel and a bodily draining passage, e.g. the urethra.

## Claims

1. A method for producing a urinary catheter (1) with parts having different characteristics, said method comprising the steps of:
- injecting at least two different fluid catheter matarials into a mould formed to define an insertable catheter trip (3) and a body (4), wherein the radial size of the tip is larger than the radial size of the rest of the catheter, and subsequently
- solidifying the material therein,

2. A method according to claim 1, wherein the catheter material is injected into the mould in more than one injection cycle.

3. A method according to claim 1 or 2, wherein the difference in the characteristics relates to the softness of the materials after solidification.

4. A method according to any of the preceding claims, wherein the difference in the characteristics relates to the colours of the material.

5. A method according to any of the preceding claims, wherein the difference in the characteristics relates to the strengths of the materials.

6. A method according to any of the preceding claims, wherein the difference in the characteristics relates the slipperiness of the materials after solidification.

7. A method according to any of the preceding claims, wherein the difference in the characteristics relates to the ability of the solidified materials to absorb liquid substances.

8. An injection moulded urinary catheter (1) with an insertable catheter tip (3) and a body (4), the catheter being made of at feast two different catheter materials, the materials being joined during the moulding process in which both materials are injected into a mould in fluid state and solidified therein, wherein the radial size of the tip is larger than the radial size of the rest of the catheter.

9. A catheter according to claim 8, comprising multiple layers, to form a laminated structure of the catheter,

10. A catheter according to any of claims 7 or 8, wherein at least one of the materials is a hydrophilic polymer material.

## Patentansprüche

1. Verfahren zur Herstellung eines Harnkatheters (1) mit Teilen mit unterschiedlichen Eigenschaften, das folgende Schritte umfasst:
- Einspritzen von mindestens zwei verschiedenen flüssigen Kathetermaterialien in eine Form, die so ausgebildet ist, dass sie eine einführbare Katheterspitze (3) und einen Körper (4) vorgibt, wobei die radiale Größe der Spitze größer ist als die radiale Größe des restlichen Katheters, und anschließend
- Verfestigen des Materials darin.

2. Verfahren nach Anspruch 1, bei dem das Kathetermaterial in mehr als einem Einspritzzyklus in die Form eingespritzt wird.

3. Verfahren nach Anspruch 1 oder 2, bei dem sich der Unterschied in den Eigenschaften auf die Weichheit der Materialien nach der Verfestigung bezieht.

4. Verfahren nach einem der vorhergehenden Ansprüche, bei dem sich der Unterschied in den Eigenschaften auf die Farben des Materials bezieht.

5. Verfahren nach einem der vorhergehenden Ansprüche, bei dem sich der Unterschied in den Eigenschaften auf die Festigkeiten der Materialien bezieht.

6. Verfahren nach einem der vorhergehenden Ansprüche, bei dem sich der Unterschied in den Eigenschaften auf die Gleitfähigkeit der Materialien nach der Verfestigung bezieht.

7. Verfahren nach einem der vorhergehenden Ansprüche, bei dem sich der Unterschied in den Eigenschaften auf die Fähigkeit der verfestigten Materialien bezieht, flüssige Substanzen zu absorbieren.

8. Durch Spritzgießen geformter Harnkatheter (1) mit einer einführbaren Katheterspitze (3) und einem Körper (4), wobei der Katheter aus mindestens zwei unterschiedlichen Kathetermaterialien hergestellt ist und die Materialien während des Formgebungsprozesses miteinander verbunden werden, bei dem die beiden Materialien in flüssigem Zustand in eine Form eingespritzt werden und sich darin verfestigten, wobei die radiale Größe der Spitze größer ist als die radiale Größe des restlichen Katheters.

9. Katheter nach Anspruch 8, der zur Erzielung einer laminierten Struktur des Katheters mehrere Schichten aufweist.

10. Katheter nach einem der Ansprüche 7 oder 8, bei dem mindestens eines der Materialien ein hydrophiles Polymermaterial ist.

## Revendications

1. Procédé de fabrication d'un cathéter urinaire (1) qui est constitué de pièces présentant des caractéristiques différentes, ledit procédé comprenant les étapes suivantes :
- injecter, à l'état fluide, au moins deux matériaux différents entrant dans la composition d'un cathéter dans un moule agencé de manière à définir un embout de cathéter insérable (3) et une partie formant corps (4), la dimension radiale de l'embout étant plus grande que la dimension radiale du reste du cathéter, et ensuite
- laisser les matériaux se solidifier dans celui-ci.

2. Procédé selon la revendication 1, dans lequel le matériau qui entre dans la composition du cathéter est injecté dans le moule en plus d'un cycle d'injection.

3. Procédé selon les revendications 1 ou 2, dans lequel la différence de caractéristiques des matériaux réside dans la douceur des matériaux après leur solidification.

4. Procédé selon l'une quelconque des revendications précédentes, dans lequel la différence de caractéristiques réside dans les couleurs des matériaux.

5. Procédé selon l'une quelconque des revendications précédentes, dans lequel la différence de caractéristiques réside dans les propriétés de résistance mécanique des matériaux.

6. Procédé selon l'une quelconque des revendications précédentes, dans lequel la différence de caractéristiques réside dans le pouvoir glissant des matériaux après leur solidification.

7. Procédé selon l'une quelconque des revendications précédentes, dans lequel la différence de caractéristiques réside dans la capacité des matériaux solidifiés à absorber des substances liquides.

8. Cathéter urinaire moulé par injection (1), comprenant un embout de cathéter insérable (3) et une partie formant corps (4), ce cathéter étant constitué d'au moins deux matériaux différents entrant dans la composition d'un cathéter, les matériaux étant assemblées lors d'un processus de moulage dans lequel les deux matériaux sont injectées dans un moule à l'état liquide et se solidifient dans celui-ci, la dimension radiale de l'embout étant plus grande que la dimension radiale du reste du cathéter.

9. Cathéter selon la revendication 8, comprenant des couches multiples de manière à conférer au cathéter une structure stratifiée.

10. Cathéter selon l'une quelconque des revendications 7 ou 8, dans lequel au moins un des matériaux est un polymère hydrophile.
